(19) Europäisches Patentamt
European Patent Office
Office européen des brevets

(11) EP 2 006 392 B1

(12) **EUROPEAN PATENT SPECIFICATION**

(45) Date of publication and mention
of the grant of the patent:
**27.03.2013 Bulletin 2013/13**

(21) Application number: **07741099.1**

(22) Date of filing: **05.04.2007**

(51) Int Cl.:
***G01N 33/68*** (2006.01)     ***C12Q 1/37*** (2006.01)

(86) International application number:
**PCT/JP2007/057663**

(87) International publication number:
**WO 2007/119685 (25.10.2007 Gazette 2007/43)**

(54) **METHOD FOR TESTING ALZHEIMER'S DISEASE BY ASSAYING DEGRADATION RATE OF beta-AMYLOID IN BLOOD AND DIAGNOSTIC REAGENT**

**TESTVERFAHREN FÜR MORBUS ALZHEIMER MIT TESTEN DER ABBAUGESCHWINDIGKEIT VON beta-AMYLOID IM BLUT UND DIAGNOSTISCHES REAGENS**

**METHODE POUR DEPISTER LA MALADIE D'ALZHEIMER PAR DOSAGE DU TAUX DE DEGRADATION DE beta-AMYLOIDE DANS LE SANG ET REACTIF DE DIAGNOSTIC**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **13.04.2006 JP 2006110881**

(43) Date of publication of application:
**24.12.2008 Bulletin 2008/52**

(73) Proprietor: **EIDIA Co., Ltd.**
**Chiyoda-ku**
**Tokyo (JP)**

(72) Inventor: **TAKAYAMA, Shigeo**
**Inashiki-gun, Ibaraki 300-1155 (JP)**

(74) Representative: **Grünecker, Kinkeldey,**
**Stockmair & Schwanhäusser**
**Leopoldstrasse 4**
**80802 München (DE)**

(56) References cited:
**WO-A1-2006/046644     JP-A- 07 051 096**

• **DU Y. ET AL.: 'alpha2-Macroglobulin as a beta-amyloid peptide-binding plasma protein'** JOURNAL OF NEUROCHEMISTRY vol. 69, no. 1, 1997, pages 299 - 305, XP002381632

• **WANG D.-S. ET AL.: 'beta-Amyloid Degradation and Alzheimer's Disease'** JOURNAL OF BIOMEDICINE & BIOTECHNOLOGY vol. 2006, no. 3, 2006, pages 1 - 12, XP008129952
• **LAUER D. ET AL.: 'Alpha 2-macroglobulin-mediated degradation of amyloid beta 1--42: a mechanism to enhance amyloid beta catabolism'** EXPERIMENTAL NEUROLOGY vol. 167, no. 2, 2001, pages 385 - 392, XP008129955
• **CRAFT S. ET AL.: 'Insulin effects on plasma beta-amyloid levels in normal adults and patients with ad'** NEUROBIOLOGY OF AGING vol. 25, no. SUPPL. 2, 2004, page S35, XP004624730
• **KOVACS D.M. ET AL.: 'alpha2-macroglobulin in late-onset Alzheimer's disease'** EXPERIMENTAL GERONTOLOGY vol. 35, no. 4, 2000, pages 473 - 479, XP008129957
• **FUKAMI S. ET AL.: 'Alzheimer's disease ni okeru Protease no Kan'yo to Rinsho Oyo'** THE TISSUE CULTURE ENGINEERING vol. 27, no. 9, 25 August 2001, pages 8 - 11
• **SAIDO T.: 'Tanpakushitsu Bunkai Hanno o Hosoku suru Atarashii Hoho o Mochiita Noikketsu oyobi Alzheimer's disease ni okeru Protease no Sayo no Kaiseki'** SEIKAGAKU vol. 68, no. 9, 25 September 1996, pages 1507 - 1522

**Description**

The present invention relates to a method for testing a disease in which β-amyloid is involved, such as Alzheimer's disease, by measuring the degradation rate of β-amyloid in a blood sample.

β-amyloid (hereinafter, referred to as "Aβ") is a main component of the amyloid plaque which is characteristic of a brain of a patient with Alzheimer's disease (AD) and it is known that Aβ is produced by a β-secretase which cleaves a β-position of an N-terminal site of a precursor protein thereof (APP) and by a γ-secretase which cleaves an APP-C-terminal site which is present in a cell membrane.

Aβ molecular species are known to have various molecular weight sizes. The best-known species of those in connection with neurotoxicity are Aβ species composed of 42 amino acids (hereinafter, referred to as "Aβ1-42"). Aβ1-42 has a nature which readily forms fibers. It is known that Aβ1-42 is deposited in the early stages of AD to form an amyloid plaque. Aβ1-42 is considered to be one of the substances which causes onset of AD.

As described above, there has been an increase of research on the amyloidogenic process of Aβ and the relationship between Aβ and onset of AD. On the other hand, in recent years, a degradation route of Aβ produced is being clarified gradually. In particular, a group of Saidou et al. has found out the presence of neprilysin, which is an Aβ-degrading enzyme localized specifically in nerve cells of the brain, and suggested that AD may be caused by increasing the level of Aβ deposited in the brain due to a decrease in the activity of the enzyme (Non-Patent Documents 1 to 3). Meanwhile, although Aβ is considered to be produced in the brain and transfered into blood via spinal fluid, it is unknown that the degradation of Aβ in a part including blood other than brain tissues, including the presence or absence of a degradation route and a degrading enzyme.

EP 0564946 discloses that the degree of proteolytic cleavage of an APP substrate in the presence of a patient's sample could be indicative for Alzheimer's disease.

Regarding diagnosis of AD, an attempt to diagnose AD patients, in which Aβ1-42 is used as a disease marker, has been investigated because Aβ1-42 is closely related to onset of AD. It has been reported that the concentration of Aβ1-42 in cerebrospinal fluid of an AD patient is reduced (Non-patent Documents 4 to 7). However, the use of cerebrospinal fluid as a sample involves a high risk of bodily impairment or bodily function damage for the patient when the sample is being collected. Thus, in practice, the use of cerebrospinal fluid as a sample is not practical at the present time, and does not come into wide use. On the other hand, it is considered that blood samples (serum or plasma) are most common as in vitro diagnostic reagents and have low risk of bodily impairment. However, it is almost impossible to detect Aβ in the serum by using the general measurement methods such as the double antibody sandwich immunoassay which has been so far carried out; hence the clinical usefulness of the Aβ measurement in the serum has not been discovered. This suggests that it is very difficult to diagnose AD patients by measuring Aβ1-42 in blood samples (Non-Patent Document 8). In a diagnostic study of AD, it is very problematic at present that diagnosis in a blood sample that is generally used as an in vitro diagnostic reagent cannot be performed.

Non-patent Document 1: Takaki Y, et al., J. Biochem(Tokyo). 2000; 128(6): 897-902.
Non-patent Document 2: Shirotani K, et al., J. Biol. Chem. 2001 276(24); 21895-901.
Non-patent Document 3: Iwata N, et al., Science. 2001 292(5521); 1550-2.
Non-patent Document 4: Tamaoka A, et al., J. Neurol. Sci. 1997; 148: 41-45.
Non-patent Document 5: Andreasen N, et al., Arch. Neurol. 1999; 56: 673-680.
Non-patent Document 6: Galasko D, et al., Arch. Neurol. 1998; 55: 937-945.
Non-patent Document 7: Motter R, et al., Ann Neurol 1995; 38: 643-648.
Non-patent Document 8: Tamaoka A, et al., J. Neurol. Sci. 1996; 141: 65-68.
Non-patent Document 9: Namba et al., Studies on New Immunoassay Method Using Electrochemiluminescence (ECL), JJCLA, 1996, Vol.21, No.5
Non-patent Document 10: Nature, 256, 459-497(1975)


Problems to be solved by the Invention

The present invention provides a method of testing Alzheimer's disease, according to claim 1 comprising measuring a β-amyloid 1-42-degrading activity in a blood sample, wherein β-amyloid 1-42 means human β-amyloid composed of 42 amino acids.

The present invention further provides the use of a diagnostic reagent for testing Alzheimer's disease according to claim 3, comprising, as essential components:

a β-amyloid 1-42 peptide to be added to a blood sample, wherein the β-amyloid 1-42 peptide is human β-amyloid composed of 42 amino acids;
an antibody which recognizes a C-terminal site of the β-amyloid 1-42 peptide; and
an antibody which recognizes an N-terminal site of the β-amyloid 1-42 peptide,

wherein the diagnostic reagent is used for measuring a residual amount of the β-amyloid 1-42 peptide added to the blood sample to measure a β-amyloid 1-42 peptide-degrading activity in the blood sample.

Preferred embodiments are set forth in the subclaims.

The inventor of the present invention has investigated whether or not the presence or absence of degradation and metabolism of an Aβ peptide in human serum could be measured with a view to resolving the above-mentioned problems. In order to confirm degradation of Aβ1-42 in blood, an Aβ1-42 synthetic peptide was added to human serum, and the residual amount of Aβ1-42 was measured with time. The measurement was performed by a double antibody sandwich immunoassay based on the electrochemiluminescence method (Non-Patent Document 9) in which an antibody specific to the N-terminal site of Aβ1-42 was used as a primary antibody and an antibody specific to the C-terminal site of Aβ1-42 was used as a secondary antibody. This method is a measurement system where it is possible to measure a full-length Aβ1-42 but impossible to measure Aβ1-42 fragments produced by degradation.

From the measurement results, it was confirmed that the measurement value of the added Aβ1-42 synthetic peptide decreases with the passage of time. Further, it was confirmed that, when the Aβ1-42 synthetic peptide was added to serum, decrease in the measurement value of the Aβ1-42 synthetic peptide with the passage of time was significantly suppressed by adding in advance a protease inhibitor capable of inhibiting the activity of the enzyme into the serum.

These facts signify that Aβ is rapidly degraded and fragmented in blood by a protease, and that measurement based on the double antibody sandwich immunoassay which enables measurement of only a full-length Aβ1-42 is impossible because the added Aβ1-42 synthetic peptide is enzymatically digested and fragmented in the blood.

Next, measurement based on the double antibody sandwich immunoassay was performed for samples obtained by adding an appropriate amount of an Aβ1-42 synthetic peptide to serum samples of AD patients and normal subject and storing the mixtures at 25°C for 20 hours. It was confirmed that, in all the samples, proteases in the samples degraded the Aβ1-42 synthetic peptide because the measurement values decrease in the serum of AD patients and normal subjects compared with those measured immediately after addition of the Aβ1-42 synthetic peptide. The results obtained on comparing the decrease rates of the measurement values in the serum of AD patients and normal subjects showed a significant difference therebetween, and the possibility of use of the serum for the diagnosis of AD was confirmed, whereby the present invention has been achieved.

That is, the method of testing Alzheimer's disease according to claim 1 is characterized by comprising measuring the Aβ-degrading activity in a blood sample. Examples of a method of measuring the Aβ-degrading activity include a method involving: adding an Aβ peptide in a blood sample; measuring the activity to degrade the peptide after a lapse of a predetermined time; and comparing the measured degrading activity with that of blood of normal subjects.

The Aβ peptide to be added to a sample is not particularly limited as long as it is a peptide which includes a site to be cleaved where the Aβ peptide in a living body is cleaved by a degradative enzyme such as neprilysin, and is a human Aβ peptide 1-42. The method of measuring the residual amount of the added Aβ peptide involves a double antibody sandwich immunoassay in which an antibody which recognizes the N-terminal site of the peptide and an antibody which recognizes the C-terminal site of the peptide. It is preferable that the above-mentioned blood sample is serum or plasma.

The AD diagnostic reagent comprises, as essential components, an Aβ peptide added to the blood sample, an antibody which recognizes the N-terminal site of the Aβ peptide, and an antibody which recognizes the C-terminal site of the Aβ peptide, and is characterized in that the activity of an Aβ peptide-degrading enzyme in a blood sample is measured by measuring the residual amount of the Aβ peptide added to the blood sample. The diagnostic reagent has a constitution which varies according to the immunoassay used and the type of the added Aβ peptide. However, in the case of using the Aβ1-42 peptide and double antibody sandwich immunoassay, the reagent may include, as components, an immobilized antibody which recognizes the N-terminal site of Aβ1-42 and an antibody which recognizes the C-terminal site of Nβ1-42 and is labeled with a labeling substance. Alternatively, the reagent may include, as components, an immobilized antibody which recognizes the C-terminal site of Aβ1-42 and an antibody which recognizes the N-terminal site of Aβ1-42 and is labeled with a labeling substance. The antibody which recognizes the C-terminal site of Aβ1-42 is not particularly limited, but it is preferably 21F12.

Both polyclonal antibodies and monoclonal antibodies can be used for the above-mentioned antibodies in the testing methods of the present invention. Among the above-mentioned antibodies, the antibody which recognizes the C-terminal site of Aβ1-42 is preferably an antibody highly specific to Aβ1-42 but not particularly limited to that. It is preferable that the above-mentioned blood sample is serum or plasma, but the sample may be whole blood. Examples of the labeling substance include fluorescent substances, enzymes, pigments, and luminescent substances and is not particularly limited, but a ruthenium complex is preferable. The support for immobilization is not particularly limited, and it is preferably magnetic beads.

A method of measuring the residual amount of an added Aβ peptide is not limited to the above-mentioned double antibody sandwich immunoassay. For example, there can be used a method involving: preparing an Aβ synthetic peptide labeled with a luminescent or fluorescent substance; adding the prepared peptide to blood; and measuring a change in the amount of luminescence or fluorescence caused by degradation of the added Aβ synthetic peptide. The essence of the present invention resides in that the Aβ-degrading activity of a composition in a blood sample can be measured according to claim 1. That is, it is a method of testing AD by measuring the activity or amount of a protease in a blood sample. The specific point of the present invention is that AD can be diagnosed by measuring the degradation rate of an Aβ peptide added to a blood sample according to claim 1, i.e., the activity of an Aβ-degrading enzyme in blood.

Another method of measuring the activity of an Aβ-degrading enzyme in blood is a method in which there is used an Aβ peptide labeled with a radioisotope, a fluorescent substance, or the like. Specifically, the method comprises; labeling a specific site of Aβ with a fluorescent substance, adding the peptide to a blood sample, and analyzing the sample by an HPLC analyzer equipped with a fluorescence detector after a lapse of a predetermined time to measure the time to elute the fluorescent substance. Degradation of an Aβ peptide can be measured by detecting a peak of a fluorescence-labeled Aβ peptide fragment, which is detected at an elution time different from that of a peak of an undegraded fluorescence-labeled Aβ peptide.

According to the present specification, it is possible to diagnose AD in a more specific manner by measuring a peak specific to AD patients even if a plurality of degradation products of the Aβ peptide are present. Meanwhile, if a peak specific to AD is specified, it is possible to specify a site specific to AD where the Aβ peptide is cleaved and to develop an AD diagnostic reagent with high specificity.

Effects of the Invention

The present invention enables AD to be diagnosed by determining the degradation degree of an Aβ synthetic peptide added to a blood sample by using the double antibody sandwich immunoassay in which an antibody which specifically recognizes an Aβ peptide is used to confirm the strength of the Aβ peptide-degrading activity of an enzyme according to the claims. According to the present invention, it is possible to diagnose AD in a blood sample such as plasma and serum.

Brief Description of the Drawings

Fig. 1 is a graph showing results of Experimental Example 1.
Fig. 2 is a graph showing results of Experimental Example 2.
Fig. 3 is a graph showing results of Example 1.
Fig. 4 is a graph showing results of Example 2.

Best Mode for carrying out the Invention

A case is described below, where using serum as a blood sample and an Aβ1-42 synthetic peptide as an Aβ peptide to be added to the serum sample, a double antibody sandwich immunoassay is adopted as a method of quantifying the peptide.

A mixture obtained by adding an Aβ1-42 synthetic peptide to a serum sample is incubated at 25°C, and a predetermined amount of the mixture is separated. Then, the residual amount of the Aβ1-42 synthetic peptide is measured by the double antibody sandwich immunoassay to calculate the peptide-degrading activity in the serum sample. The resultant value is compared with the degradation activity in serum of a normal subject to test the presence or absence of morbidity of AD. In the double antibody sandwich immunoassay, an antibody that is immobilized to magnetic beads and is specific to the N-terminal site of Aβ1-42 and an antibody that is labeled with a ruthenium complex and is specific to the C-terminal site are used, and the procedure should be carried out in accordance with a conventional method. That is, the separated sample is reacted with magnetic beads immobilized with an antibody to bind to an Aβ1-42 synthetic peptide, followed by washing (BF separation). Subsequently, a labeled secondary antibody is reacted therewith to bind to the Aβ1-42 synthetic peptide bound to the magnetic beads, and washing is performed. Finally, the ruthenium complex, which is a labeling substance bound to the magnetic beads, is allowed to emit light by applying thereto electrical energy under the presence of tryptophyl amine, whereby the amount of the ruthenium complex is measured by intensity of luminescence.

The method of measuring the residual amount of the added Nβ1-42 synthetic peptide is not particularly limited as long as the Aβ1-42 amount can be determined, and a method other than immunological methods may be employed. The antibodies to be used in the double antibody sandwich immunoassay can be either monoclonal antibodies or polyclonal antibodies as long as they are an antibody specific to the N-terminal site of Aβ1-42 and an antibody specific to the C-terminal site of Aβ1-42.

The antibody which recognizes the N-terminal site of Aβ1-42 is not limited to an antibody which recognizes the most N-terminal site, and commercially-available examples thereof include a 3D6 antibody that is an antibody which recognizes an amino acid 1-5 site of Aβ1-42 (manufactured by Innogenetics NV), a 6E10 antibody that is an antibody which recognizes an amino acid 10-16 site (manufactured by Chemicon International, Inc.), and a 4G8 antibody which recognizes an amino acid 17-24 site (manufactured by Chemicon International, Inc.).

Commercially-available examples of the antibody which recognizes the C-terminal site of Aβ1-42 include: 21F12 (manufactured by Innogenetics NV) and 8G7 (manufactured by Nanotools GmbH) which are mouse monoclonal antibodies; and AB5078P (manufactured by Chemicon International, Inc.) which is a rabbit polyclonal antibody.

It is also possible to employ a method of measuring the degradation rate of an Aβ1-40 synthetic peptide in a blood sample by the double antibody sandwich immunoassay system in which there are used an antibody which recognizes the C-terminal site of Aβ1-40 and an antibody which recognizes the N-terminal site thereof. In the case where a 6E10 antibody which recognizes an amino acid 10-16 site is used as an antibody which recognizes the N-terminal site, it is possible to shorten the N-terminal site of an Aβ synthetic peptide. Moreover, if an antibody which recognizes a degradation (cleavage) site of an Aβ synthetic peptide is used, the presence or absence of degradation in a sample can be determined. In the case where an immunoassay is employed as a method of measuring the peptide, the length of a peptide can be selected appropriately in view of the specificity or the like of a recognition site of an antibody to be used. In the method of the present invention, an Aβ1-42 synthetic peptide to be used as a substrate may be any of peptides manufactured by different manufacturers as long as the peptide has the same amino acid sequence as that of Aβ1-42 from the human. The peptides can be synthesized by the usual method such as a solid phase synthetic method.

Materials for a support to immobilize the antibody may be glass, plastic (for example, polystyrene, polyamide, polyethylene, or polypropylene), metal, or the like. The support may take a form of a cup, a flat plate, particles, or the like with no particular limitation. It is preferable that the materials be magnetic microbeads (magnetic beads).

The immobilization of the antibody onto the support is performed according to the usual method. In a case where the magnetic beads are used as the support, the following process is preferable; magnetic beads and the antibody are reacted in a buffer solution, after that, the magnetic beads are treated with a blocking agent, and preserved in the blocking agent until they are used.

The labeling substance to be used in the present invention is not limited to an enzyme, a luminous substance, a fluorescent substance, an isotope, and a ruthenium complex is preferable. The method of preparing the labeling antibody is carried out in accordance with the usual methods. For example, the antibody and a ruthenium complex (Origen TAG-NHS Ester, manufactured by Igen Co.) are reacted in a buffer solution, and then, 2M of glycine is added thereto to cause further reaction. After that, the preparation can be achieved by purifying the labeled antibody by gel filtration column chromatography.

A diagnostic reagent (kit) based on the double antibody sandwich immunoassay comprises, as essential components, an antibody specific to the N-terminal site of Aβ1-42, an antibody specific to the C-terminal site thereof, and an Aβ1-42 synthetic peptide. Note that the reagent may be freely accompanied by a buffer, a measurement tool, or the like for the reaction. As described above, an Aβ synthetic peptide to be added to a sample can be selected appropriately depending on the specificity of an antibody to be used.

The antibodies used in the present invention can be prepared using the usual methods. The preparation of a monoclonal antibody involves using a peptide which retains the C-terminal site of Aβ1-42 as antigen, producing a composite thereof with a carrier protein as required, and inoculating the composite into an animal to immunize it. The antibody forming cells obtained from the spleen or lymph nodes of the above-mentioned immunized animal are fused with myeloma cells and prepared by selecting the hybridomas which produce antibodies which exhibit strong specificity to the C-terminal site of Aβ1-42. The procedure should be carried out in accordance with the existing known methods. Aβ1-42 can also be used as the immunogen, but since the target antibodies are antibodies which are specific to the C-terminal site of Aβ1-42, peptides each of which retains the C-terminal site of Aβ1-42, such as Aβ33-42, can be selected appropriately. Generally, a composite of the immunogen and a carrier protein is used for the antigen, and the composite can be prepared using various condensing agents, such as glutaraldehyde, carbodiimide, and maleimide active esters. Bovine serum albumin, thyroglobulin, hemocyanin, and the like can be used for the carrier protein and methods involving coupling in a proportion of 1 to 5 times are generally used.

The animal which is immunized with the immunogen may be a mouse, a guinea pig or the like and the inoculation can be carried out subcutaneously, intramuscularly, or intraperitoneally. In administration of the immunogen, it may be administered as a mixture with complete Freund's adjuvant or incomplete Freund's adjuvant, and the administration is generally carried out once per 2 to 5 weeks. The antibody producing cells obtained from the spleen or lymph nodes of the immunized animal are formed into fused cells with myeloma cells and isolated as hybridomas. Myeloma cells originating from mice, rats, humans, or the like, can be used for the myeloma cells, and cells originating from the same species as the antibody producing cells are preferable, but there are cases where cells originating from different species can be used.

The cell fusion operation can be carried out using such a known method as the Köhler and Milstein method (Non-Patent Document 10). There can be mentioned polyethylene glycol, Sendai virus or the like as fusion promoting agents. The cell fusion can be carried out generally by reacting the antibody producing cells and the myeloma cells in a ratio of about 1:1 to 1:10 for a period of about 1 to 10 minutes using a concentration of about 20 to 50% of polyethylene glycol (average molecular weight 1,000 to 4,000) at a temperature of 20 to 40°C, and preferably of 30 to 37°C.

The screening for the hybridomas which produce antibodies which have specificity to the C-terminal site of Aβ1-42 can be carried out using various immunochemical methods. For example, the ELISA method, the Western Blot method, or the competitive method can be employed. Further, antibodies which react with Aβ1-42 and do not react with Aβ1-40 can be selected by using the Aβ1-42 peptide and the Aβ1-40 peptide.

Cloning is then carried out by means of the limiting dilution method, for example, from the wells which have been selected in this way and the target clones can be obtained. Generally, the selection and growth of the hybridomas is carried out in an animal cell culture medium (RPMI1640, for example) to which HAT (hypoxanthine, aminopterin, and thymidine) has been added and which contains from 10 to 20% bovine fetal serum. The clones obtained in this way are transplanted into the abdominal cavities of a BALB/C mouse to which Britstan has been administered beforehand, and abdominal fluid which contains a high concentration of the monoclonal antibody is collected after 10 to 14 days and this can be used as the raw material for antibody purification. Further, the clones can be cultured and the culture liquid can be used as the raw material for antibody purification. The recovery of the monoclonal antibodies should be achieved using a known method for the purification of immunoglobulins. For example, it can be achieved by means such as ammonium sulfate fractionation, PEG fractionation, and ethanol fractionation, using an anion exchange material, or using affinity chromatography.

Polyclonal antibodies can also be prepared using the usual methods. They can be prepared as a composite by immunizing an animal such as a rabbit or guinea pig with the same procedure as described above using a peptide a principal structure of which is the C-terminal site of Aβ1-42 as an antigen. Polyclonal antibodies can be obtained through purification by the methods described above with measuring the antibody potency after appropriate collection of blood and using the serum with the high potency as the raw material for the purification of the antibody.

A method of diagnosing AD by measuring the degradation rate of an Aβ synthetic peptide added to a blood sample (plasma, serum) based on the double antibody sandwich immunoassay, is excellent and usable for AD diagnosis in a blood sample. An in vitro diagnostic reagent to be used for AD diagnosis in which a blood sample is used has not been developed, and no report has been made on an attempt to diagnose AD by measuring the degradation degree of Aβ1-42 with focusing an Aβ-degrading enzyme in blood. Therefore, the inventor of the present invention first discovered that the method according to claim 1 is useful for AD diagnosis. The present specification proves that the clinical availability in blood samples of AD patients and normal subjects by measuring degradation of an Aβ peptide and enables diagnosis in a blood sample for AD. A method of measuring an Aβ-degrading enzyme may be employed, and used for diagnosis of a disease caused by Aβ or the like.

Examples

Hereinafter, the present invention is described in detail by means of examples, but the present invention is not limited to the illustrative examples described below as long as there is no deviation from the essential features of the present invention.

(Experimental Example 1) Electrochemiluminescence double antibody sandwich immunoassay in which a 3D6 antibody and a 21F12 antibody are used:

A 3D6 mouse monoclonal antibody (manufactured by Innogenetics NV) which was an antibody which recognizes a 1-5 amino acid site of Aβ1-42 was used as a primary antibody, and a 21F12 mouse monoclonal antibody (manufactured by Innogenetics NV) which was a C-terminal site recognition antibody of Aβ1-42 was labeled with a ruthenium complex and used as a secondary antibody.

The methods used to prepare the respective constitutional components of a reagent are described hereinbelow.

(1) Method of preparing 3D6 antibody-binding magnetic beads:

A 3D6 mouse monoclonal antibody was diluted to an antibody concentration of 1 mg/mL with a 10 mmol/L potassium phosphate buffer solution (pH 7.8), and 0.5 mL of the antibody was mixed with 0.5 mL of magnetic beads (DYNABEADS M-450 Epoxy, manufactured by Dynal Co.) having a concentration of 30 mg/mL. The liquid mixture was stirred for 16 hours at 25°C so that the antibody was bound to the magnetic beads. Then, only the liquid solution was removed from the magnetic bead solution to remove free antibodies which had not been bound to the magnetic beads and remained in the liquid solution. Then, 1 mL of a 4% Block Ace reagent (manufactured by Dainippon Pharmaceutical Co., Ltd.) as a blocking agent was added to the antibody-binding magnetic beads, and the mixture was stirred for 3 hours at 25°C. Then, the magnetic beads were washed with 10 mL of the 4% Block Ace reagent (washing five times with 2 mL of the 4% Block Ace reagent). After washing, the 3D6 antibody-binding magnetic beads were mixed with 0.5 mL of the 4% Block Ace reagent and stored at 4°C until they were to be used.

(2) Method of preparing ruthenium complex-labeled 21F12 antibody:

A 21F12 mouse monoclonal antibody (manufactured by Innogenetics NV) was diluted to an antibody concentration of I mg/mL with a 10 mmol/L potassium phosphate buffer solution (pH 7.8). Then, 17.6 μl of a 10 mg/mL ruthenium complex (Origen TAG-NHS Ester, manufactured by Igen Co.) was added to 0.5 mL of the 1 mg/mL antibody, and the mixture was stirred for 30 minutes at 25°C. After that, 30 μL of 2 mol/L glycine was added thereto, and the mixture was stirred for 30 minutes at 25°C.

Next, the ruthenium-labeled antibody solution was applied to gel filtration column chromatography (Sephadex G-25, manufactured by Amersham Biosciences K.K.) packed into a glass tube of diameter 1 cm and height 30 cm, and the ruthenium-labeled antibodies were isolated from the non-labeled ruthenium and purified. Elution was carried out with a 10 mmol/L potassium phosphate buffer solution (pH 6.0).

(3) Measurement of Aβ1-42 peptide by double antibody sandwich immunoassay:

The required number of 500 μL polystyrene cups (hereinafter, referred to as "reaction cups") were prepared, and 200 μL of a reaction solution containing 50 mmol/L MOPS, 1% (w/v) Block Ace (manufactured by Dainippon Pharmaceutical Co., Ltd.), 0.15 mol/L NaCl, 0.01% (w/v) Tween 20™, 10 mmol/L EDTA2Na, and 0.1 % CHAPS (pH 7.2) (hereinafter, referred to as "reaction solution") was poured into each of the reaction cups. 20 μL of a sample obtained by diluting the Aβ1-42 synthetic peptide (manufactured by Peptide Institute, Inc.), the Aβ1-40 synthetic peptide (manufactured by Peptide Institute, Inc.), the Aβ1-11 synthetic peptide (manufactured by Behem), and the Aβ34-42 synthetic peptide (manufactured by Sigma-Aldrich Japan K.K.) with the reaction solution to a concentration of 0, 1, 5, 10, 50, 100, 200 or 400 pg/mL was added to each of those reaction cups. Subsequently, 25 μL of 3D6 antibody-binding magnetic beads which had been diluted to a concentration of 1 mg/mL with the reaction solution was added to each of the reaction cups to allow a reaction to proceed for 9 minutes at 30°C (first reaction).

Subsequently, the magnetic beads were trapped with a magnet and the liquid was removed from the reaction cups. The magnetic beads were washed twice with 350 μL of 50 mmol/L Tris HCl, Tween 20, and 0.15 mol/L NaCl (pH 7.5) (hereinafter, referred to as "washing solution"), and non-specifically-binding substances other than that of the antigen-antibody reaction was removed. Then, 200 μL of a ruthenium-labeled 21F12 antibody which had been diluted to a concentration of 4 μg/mL with the reaction solution was added and reacted for 9 minutes at 30°C (second reaction). After the reaction, the magnetic beads were trapped with a magnet, and the liquid in the cups was removed. The magnetic beads were washed twice with 350 μL of a washing solution and the non-specifically-binding substances other than that of the antigen-antibody reaction were removed.

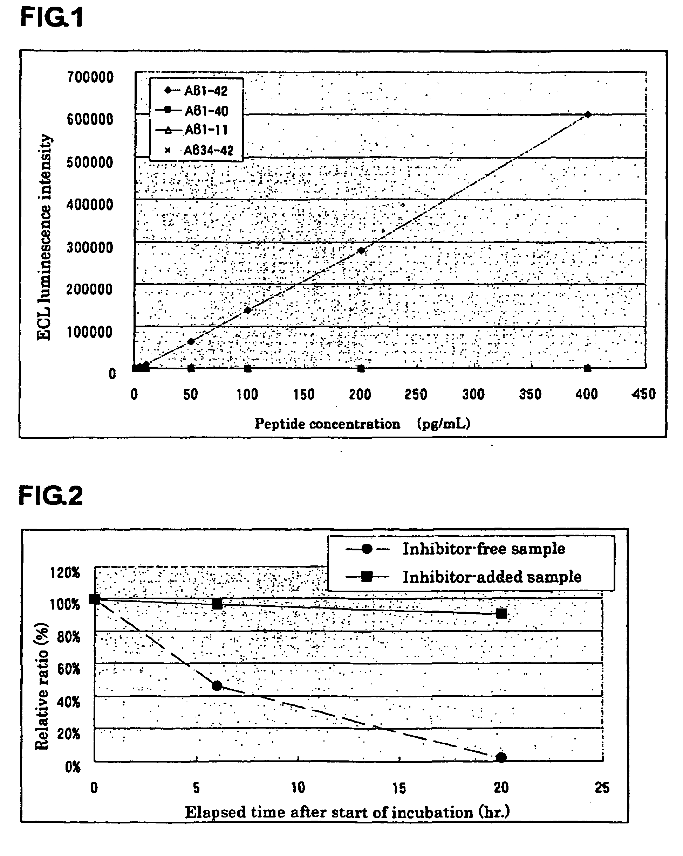

Subsequently, 300 μL of tryptophyl amine which is a luminescent substrate was put into each cup, and mixed with the magnetic beads. The ruthenium emitted light when electrical energy was applied in this state, and the luminescence intensity was detected with a detector. Incidentally, the measuring procedure after the addition of the magnetic beads to the reaction cups described above was carried out with an automated ruthenium luminescence measurement device Picolumi 8220 (manufactured by Sanko Junyaku Co., Ltd.). Table 1 and Fig. 1 show the results.

[Table 1]

| Results of Experimental Example 1 | | | |
|---|---|---|---|
| Sample | Sample concentration (pg/mL) | ECL luminescence intensity | Difference from the Blank |
| Blank | 0 | 286.1 | - |
| Aβ1-42 peptide | 1 | 771.0 | 484.9 |
| | 5 | 4124.5 | 3838.4 |
| | 10 | 9027.1 | 8741.0 |
| | 50 | 62800.0 | 62513.9 |
| | 100 | 138701.6 | 138415.5 |
| | 200 | 281159.2 | 280873.1 |
| | 400 | 600815.1 | 600529.0 |

(continued)

| Results of Experimental Example 1 | | | |
|---|---|---|---|
| Sample | Sample concentration (pg/mL) | ECL luminescence intensity | Difference from the Blank |
| Aβ1-40 peptide | 1 | 302.1 | 16.0 |
| | 5 | 297.8 | 11.7 |
| | 10 | 280.3 | -5.8 |
| | 50 | 311.2 | 25.1 |
| | 100 | 290.1 | 4.0 |
| | 200 | 287.9 | 1.8 |
| | 400 | 307.9 | 21.8 |
| Aβ1-11 peptide | 1 | 267.5 | -18.6 |
| | 5 | 296.1 | 10.0 |
| | 10 | 288.3 | 2.2 |
| | 50 | 367.1 | 81.0 |
| | 100 | 290.4 | 4.3 |
| | 200 | 311.0 | 24.9 |
| | 400 | 291.1 | 5.0 |
| Aβ34-42 peptide | 1 | 302.1 | 16.0 |
| | 5 | 277.1 | -9.0 |
| | 10 | 293.7 | 7.6 |
| | 50 | 295.0 | 8.9 |
| | 100 | 290.2 | 4.1 |
| | 200 | 298.1 | 12.0 |
| | 400 | 287.3 | 1.2 |

As shown in Table 1 and Fig. 1, the double antibody sandwich immunoassay was confirmed to be a measurement system which enabled specific detection of only a full-length Aβ1-42 peptide but disabled detection of an Aβ fragment.

(Experimental Example 2) Test for confirming degradation of Aβ1-42 synthetic peptide by enzyme in serum:

Serum of a normal subject was dispensed into two microtubes (Immuno Ware Micro Tubes manufactured by Pierce, hereinafter, referred to as "microtubes") in an amount of 90 μL each. Then, 9 μL of protease inhibitor cocktail (manufactured by Roche Diagnostics K. K.) was added to one of the two microtubes (hereinafter, referred to as "inhibitor-added sample"), while 9 μL of sterilized water was added to the other microtube (hereinafter, referred to as "inhibitor-free sample"). Then, the tubes were incubated at 25°C.
Immediately after preparation of the samples (0 hours), 6 hours after the start of incubation, and 20 hours after the start of incubation, a 10-μL portion of each sample was sampled and mixed with 200 μL of a reaction solution in a microtube (hereinafter, referred to as "pre-measurement diluted sample"). Measurement of the pre-measurement diluted samples was performed immediately after the preparation by the Aβ1-42 sandwich immunoassay in the same way as Experimental Example 1.
The results of the measurement of ECL luminescence intensities are shown in Table 2. In Table 2, the measured values for the samples are shown as relative ratios taking the measured values immediately after preparation of the samples to be 100%. Fig. 2 is a graph showing the relative ratios.
[Table 2]

| Results of Experimental Example 2 | | | | |
|---|---|---|---|---|
| Sample | Elapsed time after start of incubation | ECL luminescence intensity | Difference from the Blank | Relative ratio % |
| Blank<br>Inhibitor-free sample<br>Inhibitor-added sample | 0 hours | 295.2<br>201217.2<br>208443.8 | -<br>200922.0<br>208148.6 | -<br>100<br>100 |
| Blank<br>Inhibitor-free sample<br>Inhibitor-added sample | 6 hours | 280.9<br>91732.7<br>201843.9 | -<br>91451.8<br>201563.0 | -<br>46<br>97 |
| Blank<br>Inhibitor-free sample<br>Inhibitor-added sample | 20 hours | 277.8<br>3850.1<br>188281.8 | -<br>3572.3<br>188004.0 | -<br>2<br>90 |

As shown in Table 2 and Fig. 2, the serum to which the Aβ1-42 synthetic peptide had been added after the addition of the protease inhibitor provided results that the reduction in the measured value with the passage of time had clearly been suppressed. Therefore, it is considered that the measured values were reduced with the passage of time in the double antibody sandwich immunoassay system which enabled detection of only a full-length Aβ1-42 peptide, because the serum contains an enzyme capable of degrading Aβ1-42 and the added Aβ1-42 synthetic peptide is degraded by the enzyme into fragments with the passage of time. On the other hand, the results suggested that, in the case where a protease inhibitor was added, the residual amount of Aβ1-42 was larger compared with the case where no protease inhibitor was added, thereby suppressing a reduction in the measured value, because degradation of Aβ1-42 by the enzyme was inhibited.

(Example 1) Comparison of degradation rate of Aβ1-42 in serum of AD patients and normal subjects:

Into a required number of microtubes, serum of each of 30 AD patients and serum of each of 30 normal subjects were dispensed in an amount of 100 μL each. 1 μL of a 400 ng/mL Aβ1-42 synthetic peptide was added to the dispensed samples. At the same time, a standard substance was prepared by adding 1 μL of a 400 ng/mL Aβ1-42 synthetic peptide to 100 μL of a reaction solution instead of a serum sample. The Aβ1-42 synthetic peptide-added serum and standard substance were incubated at 25°C for 20 hours.
20 hours after start of incubation, a 10 μL portion was sampled from each sample and mixed with 200 μL of a reaction solution in a microtube, to thereby produce a pre-measurement diluted sample. Measurement of the pre-measurement diluted samples was performed immediately after the preparation by the double antibody sandwich immunoassay in the same way as Experimental Example 1.
The measurement results of ECL luminescence intensities of the AD patients are shown in Table 3, and the measurement results of ECL luminescence intensities of the normal subjects are shown in Table 4. In Tables 3 and 4, the relative ratio of a luminescence intensity of each sample is represented as a residual rate regarding the luminescence intensity of the standard substance as 100%. Moreover, the tables show the degradation rates of Aβ1-42 (%), calculated from the residual rates. The degradation rate is calculated by the following equation (1).

$$\text{Degradation rate} = (1 - \text{luminescence intensity of sample} \div \text{luminescence intensity of standard substance}) \times 100 \cdots (1)$$

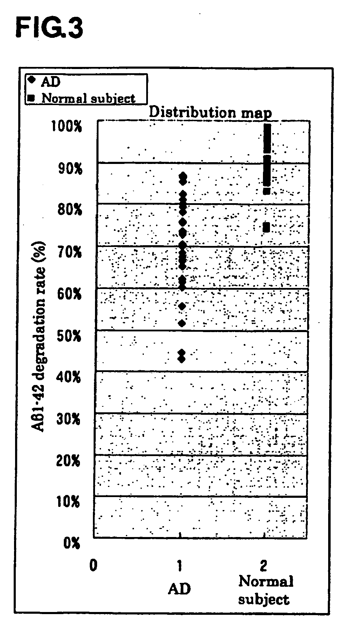

Further, Tables 3 and 4 show average values of the degradation rates for the AD patients and normal subjects, respectively. Fig. 3 is a graph showing the Aβ1-42 degradation rates of the AD patients and normal subjects.
[Table 3]

**Results of samples of AD patients in Example 1**

| Sample | ECL luminescence intensity | Difference from the Blank | Residual rate (%) | Degradation rate (%) | Average value (%) |
|---|---|---|---|---|---|
| Blank | 283.5 | - | - | - | - |
| Standard substance | 185636.1 | 185352.6 | 100 | 0 | - |
| AD patients | | | | | |
| 1 | 58851.8 | 58568.3 | 32 | 68 | |
| 2 | 71864.7 | 71581.2 | 39 | 61 | |
| 3 | 64720.8 | 64437.3 | 35 | 65 | |
| 4 | 82682.7 | 82399.2 | 44 | 56 | |
| 5 | 45090.5 | 44807.0 | 24 | 76 | |
| 6 | 60320.9 | 60037.4 | 32 | 68 | |
| 7 | 64796.5 | 64513.0 | 35 | 65 | |
| 8 | 33154.1 | 32870.6 | 18 | 82 | |
| 9 | 27314.6 | 27031.1 | 15 | 85 | |
| 10 | 49100.1 | 48816.6 | 26 | 74 | |
| 11 | 38677.8 | 38394.3 | 21 | 79 | |
| 12 | 73834.7 | 73551.2 | 40 | 60 | |
| 13 | 55765.3 | 55481.8 | 30 | 70 | |
| 14 | 55550.0 | 55266.5 | 30 | 70 | |
| 15 | 40748.3 | 40464.8 | 22 | 78 | 69 |
| 16 | 70231.4 | 69947.9 | 38 | 62 | |
| 17 | 49873.6 | 49590.1 | 27 | 73 | |
| 18 | 102935.4 | 102651.9 | 55 | 45 | |
| 19 | 62030.9 | 61747.4 | 33 | 67 | |
| 20 | 105944.0 | 105660.5 | 57 | 43 | |
| 21 | 54783.9 | 54500.4 | 29 | 71 | |
| 22 | 82510.7 | 82227.2 | 44 | 56 | |
| 23 | 50645.4 | 50361.9 | 27 | 73 | |
| 24 | 62341.1 | 62057.6 | 33 | 67 | |
| 25 | 37180.3 | 36896.8 | 20 | 80 | |
| 26 | 45537.1 | 45253.6 | 24 | 76 | |
| 27 | 24548.2 | 24264.7 | 13 | 87 | |
| 28 | 35322.8 | 35039.3 | 19 | 81 | |
| 29 | 25619.2 | 25335.7 | 14 | 86 | |
| 30 | 90013.5 | 89730.0 | 48 | 52 | |

[Table 4]

| Results of samples of normal subjects in Example 1 | | | | | |
|---|---|---|---|---|---|
| Sample | ECL luminescence intensity | Difference from the Blank | Residual rate (%) | Degradation rate (%) | Average value (%) |
| Blank | 283.5 | - | - | - | - |
| Standard substance | 185636.1 | 185352.6 | 100 | 0 | - |

(continued)

| Results of samples of normal subjects in Example 1 | | | | | |
|---|---|---|---|---|---|
| Sample | ECL luminescence intensity | Difference from the Blank | Residual rate (%) | Degradation rate (%) | Average value (%) |
| Normal subjects | | | | | |
| 1 | 8327.6 | 8044.1 | 4 | 96 | |
| 2 | 13559.4 | 13275.9 | 7 | 93 | |
| 3 | 2894.7 | 2611.2 | 1 | 99 | |
| 4 | 21526.1 | 21242.6 | 11 | 89 | |
| 5 | 6212.5 | 5929.0 | 3 | 97 | |
| 6 | 19143.9 | 18860.4 | 10 | 90 | |
| 7 | 31790.3 | 31506.8 | 17 | 83 | |
| 8 | 46978.0 | 46694.5 | 25 | 75 | |
| 9 | 24580.2 | 24296.7 | 13 | 87 | |
| 10 | 17087.3 | 16803.8 | 9 | 91 | |
| 11 | 19019.9 | 18736.4 | 10 | 90 | |
| 12 | 11035.9 | 10752.4 | 6 | 94 | |
| 13 | 48559.0 | 48275.5 | 26 | 74 | |
| 14 | 3850.1 | 3566.6 | 2 | 98 | |
| 15 | 8402.4 | 8118.9 | 4 | 96 | 89 |
| 16 | 18535.3 | 18251.8 | 10 | 90 | |
| 17 | 26507.6 | 26224.1 | 14 | 86 | |
| 18 | 25285.1 | 25001.6 | 13 | 87 | |
| 19 | 24973.5 | 24690.0 | 13 | 87 | |
| 20 | 31763.9 | 31480.4 | 17 | 83 | |
| 21 | 13458.4 | 13174.9 | 7 | 93 | |
| 22 | 46984.2 | 46700.7 | 25 | 75 | |
| 23 | 11783.4 | 11499.9 | 6 | 94 | |
| 24 | 27980.4 | 27696.9 | 15 | 85 | |
| 25 | 22642.8 | 22359.3 | 12 | 88 | |
| 26 | 24479.0 | 24195.5 | 13 | 87 | |
| 27 | 4375.3 | 4091.8 | 2 | 98 | |
| 28 | 31787.1 | 31503.6 | 17 | 83 | |
| 29 | 19323.7 | 19040.2 | 10 | 90 | |
| 30 | 19570.9 | 19287.4 | 10 | 90 | |

As shown in Tables 3 and 4 and Fig. 3, the $A\beta1$-42 degradation rates of the group of normal subjects were confirmed to be higher than those of the group of AD patients. This suggests that the ability to degrade $A\beta1$-42 in blood of the group of normal subjects is higher than that of the group of AD patients. In addition, it was determined by the t-test that there was a significant difference between the two groups ($p < 0.01$), and the result reveals that the method of the present invention can be used for AD diagnosis using serum samples.

(Example 2) Comparison of degradation rate of $A\beta1$-42 in serum of an AD patient and a normal subject by electrochemiluminescence double antibody immunoassay in which a 6E10 antibody and a 21F12 antibody are used:

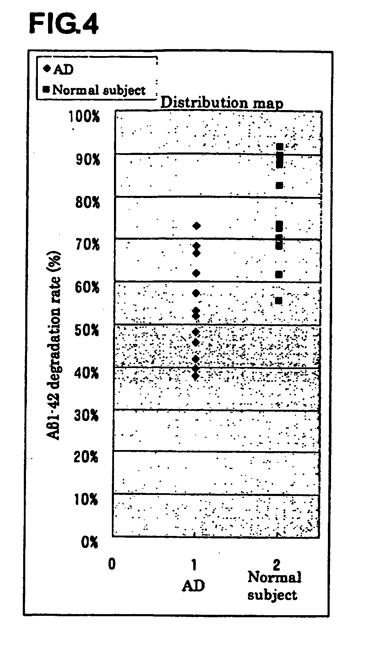

A 6E10 mouse monoclonal antibody (manufactured by Chemicon International, Inc.) which recognizes a 10-16 amino acid site of $A\beta1$-42 was used as a primary antibody, and a 21F12 mouse monoclonal antibody (manufactured by Innogenetics NV) which recognizes the C-terminal site of $A\beta1$-42 and was labeled with ruthenium complex used as a secondary antibody. The method of preparing 6E10 antibody-binding magnetic beads, method of preparing the 21F12 antibody labeled with ruthenium complex, and method of double antibody sandwich immunoassay were the same as those in Experimental Example 1. The results are shown in Tables 5 and 6 and Fig. 4.

[Table 5]

| Results of samples of AD patients in Example 2 | | | | | |
|---|---|---|---|---|---|
| Sample | ECL luminescence intensity | Difference from the Blank | Residual rate (%) | Degradation rate (%) | Average value |
| Blank | 199.8 | | - | - | (%) |
| Standard substance | 48543.4 | 48343.7 | 100 | 0 | |
| AD patients | | | | | |
| 31 | 20766.9 | 20567.2 | 43 | 57 | |
| 32 | 23410.7 | 23211.0 | 48 | 52 | |
| 33 | 20757.9 | 20558.2 | 43 | 57 | |
| 34 | 18542.0 | 18342.3 | 38 | 62 | |
| 35 | 16370.8 | 16171.1 | 33 | 67 | |
| 36 | 18571.4 | 18371.7 | 38 | 62 | |
| 37 | 13287.9 | 13088.2 | 27 | 73 | 54 |
| 38 | 26418.2 | 26218.5 | 54 | 46 | |
| 39 | 26393.8 | 26194.1 | 54 | 46 | |
| 40 | 29402.9 | 29203.2 | 60 | 40 | |
| 41 | 15522.0 | 15322.3 | 32 | 68 | |
| 42 | 28302.5 | 28102.8 | 58 | 42 | |
| 43 | 25235.2 | 25035.5 | 52 | 48 | |
| 44 | 22783.9 | 22584.2 | 47 | 53 | |
| 45 | 30225.6 | 30025.9 | 62 | 38 | |

[Table 6]

| Results of samples of normal subjects in Example 2 | | | | | |
|---|---|---|---|---|---|
| Sample | ECL luminescence intensity | Difference from the Blank | Residual rate (%) | Degradation rate (%) | Average value (%) |
| Blank | 199.8 | - | - | - | - |
| Standard substance | 48543.4 | 48343.7 | 100 | 0 | - |
| Normal subjects | | | | | |
| 31 | 14591.8 | 14392.1 | 30 | 70 | |
| 32 | 5206.2 | 5006.5 | 10 | 90 | |
| 33 | 15326.9 | 15127.2 | 31 | 69 | |
| 34 | 12944.4 | 12744.7 | 26 | 74 | |
| 35 | 13526.9 | 13327.2 | 28 | 72 | |
| 36 | 18627.1 | 18427.4 | 38 | 62 | |
| 37 | 5636.7 | 5437.0 | 11 | 89 | 76 |
| 38 | 21588.1 | 21388.4 | 44 | 56 | |
| 39 | 13439.9 | 13240.2 | 27 | 73 | |
| 40 | 8579.5 | 8379.8 | 17 | 83 | |
| 41 | 15473.5 | 15273.8 | 32 | 68 | |

(continued)

| Results of samples of normal subjects in Example 2 | | | | | |
|---|---|---|---|---|---|
| Sample | ECL luminescence intensity | Difference from the Blank | Residual rate (%) | Degradation rate (%) | Average value (%) |
| 42 | 14528.0 | 14328.3 | 30 | 70 | |
| 43 | 6204.2 | 6004.5 | 12 | 88 | |
| 44 | 8663.9 | 8464.2 | 18 | 82 | |
| 45 | 4189.1 | 3989.4 | 8 | 92 | |

As shown in Tables 5 and 6 and Fig. 4, the Aβ1-42 degradation rates of the group of normal subject were confirmed to be higher than those of the group of AD patients. As in Example 1, it was determined by the t-test that there was a significant difference between the two groups, that is, a group of the AD patients and a group of the normal subjects ($p < 0.01$). This reveals that diagnosis of AD can be performed by sandwich assay in which the 6E10 antibody which recognizes a 10-16 amino acid site was used instead of the 3D6 antibody which recognizes a 1-5 amino acid site of the N-terminal of Aβ1-42.

Industrial Applicability

The method according to claim 1, which enables measurement of an Aβ1-42-degrading enzyme activity in blood, can be used for diagnosis of Alzheimer's disease in a blood sample (serum, plasma).

**Claims**

1. A method of testing Alzheimer's disease, said method comprising
   adding a β-amyloid 1-42 peptide to a blood sample, wherein β-amyloid 1-42 means human β-amyloid composed of 42 amino acids and wherein the β-amyloid 1-42 peptide includes a site to be cleaved by a degradative enzyme, measuring a residual amount of the β-amyloid 1-42 peptide added to the blood sample by a double antibody sandwich immunoassay, wherein an antibody which recognizes a C-terminal site of the β-amyloid 1-42 peptide to be added to the blood sample and an antibody which recognizes an N-terminal site thereof are used, to measure a β-amyloid 1-42 peptide-degrading activity in the blood sample, and
   comparing the measured degrading activity with that of blood of normal subjects.

2. The method of testing Alzheimer's disease according to claim 1, wherein the blood sample is serum or plasma.

3. Use of a diagnostic reagent for testing Alzheimer's disease, comprising, as essential components:

   a β-amyloid 1-42 peptide to be added to a blood sample, wherein the β-amyloid 1-42 peptide is human β-amyloid composed of 42 amino acids and wherein the β-amyloid 1-42 peptide includes a site to be cleaved by a degradative enzyme;
   an antibody which recognizes a C-terminal site of the β-amyloid 1-42 peptide; and
   an antibody which recognizes an N-terminal site of the β-amyloid 1-42 peptide,
   wherein the antibody which recognizes a C-terminal site of the β-amyloid 1-42 peptide and the antibody which recognizes an N-terminal site of the β-amyloid 1-42 peptide are used for measuring a residual amount of the β-amyloid 1-42 peptide added to the blood sample to measure a β-amyloid 1-42 peptide-degrading activity in the blood sample.

4. The use according to claim 3, wherein the blood sample is serum or plasma.

**Patentansprüche**

1. Verfahren zum Testen der Alzheimer-Erkrankung, wobei das Verfahren umfasst
   Zugeben eines β-Amyloid-1-42-Peptids zu einer Blutprobe, wobei β-Amyloid-1-42-Peptid menschliches, aus 42 Aminosäuren bestehendes β-Amyloid bedeutet und wobei das β-Amyloid-1-42-Peptid eine Stelle einschließt, die durch ein abbauendes Enzym gespalten werden soll,
Messen einer Restmenge des zu der Blutprobe zugegebenen β-Amyloid-1-42-Peptids durch einen Doppel-Antikörper-Sandwich-Immunassay, wobei ein Antikörper, der eine C-terminale Stelle des zu der Blutprobe zuzugebenden β-Amyloid-1-42-Peptids erkennt, und ein Antikörper, der eine N-terminale Stelle desselben erkennt, verwendet werden, um eine das β-Amyloid-1-42-Peptid abbauende Aktivität in der Blutprobe zu messen, und
Vergleichen der gemessenen abbauenden Aktivität mit der von Blut normaler Probanden.

2. Verfahren zum Testen der Alzheimer-Erkrankung gemäß Anspruch 1, wobei es sich bei der Blutprobe um Serum oder Plasma handelt.

3. Verwendung eines diagnostischen Reagens zum Testen der Alzheimer-Erkrankung, umfassend als unerlässliche Bestandteile:

ein zu der Blutprobe zuzugebendes β-Amyloid-1-42-Peptid, wobei es sich bei dem β-Amyloid-1-42-Peptid um menschliches, aus 42 Aminosäuren bestehendes β-Amyloid handelt und wobei das β-Amyloid-1-42-Peptid eine Stelle einschließt, die durch ein abbauendes Enzym gespalten werden soll;
einen Antikörper, der eine C-terminale Stelle des β-Amyloid-1-42-Peptids erkennt, und
einen Antikörper, der eine N-terminale Stelle des β-Amyloid-1-42-Peptids erkennt,
wobei der Antikörper, der eine C-terminale Stelle des β-Amyloid-1-42-Peptids erkennt, und der Antikörper, der eine N-terminale Stelle des β-Amyloid-1-42-Peptids erkennt, zum Messen einer Restmenge des zu der Blutprobe zugegebenen β-Amyloid-1-42-Peptids verwendet werden, um eine das β-Amyloid-1-42-Peptid abbauende Aktivität in der Blutprobe zu messen.

4. Verwendung gemäß Anspruch 3, wobei es sich bei der Blutprobe um Serum oder Plasma handelt.

## Revendications

1. Méthode de dépistage de la maladie d'Alzheimer, ladite méthode comprenant
l'ajout d'un peptide ß-amyloide 1-42 à un échantillon de sang, où ß-amyloide 1-42 signifie ß-amyloide humain composé de 42 acides aminés et où le peptide ß-amyloïde 1-42 contient un site devant être clivé par une enzyme de dégradation,
la mesure d'une quantité résiduelle du peptide ß-amyloïde 1-42 ainsi ajouté à l'échantillon de sang par un immunodosage de type sandwich à deux anticorps, où l'on utilise un anticorps qui reconnaît un site C-terminal du peptide ß-amyloïde 1-42 devant être ajouté à l'échantillon de sang et un anticorps qui reconnaît un site N-terminal de celui-ci, pour mesurer une activité de dégradation du peptide ß-amyloïde 1-42 dans l'échantillon de sang, et
la comparaison de l'activité de dégradation ainsi mesurée à celle du sang de sujets normaux.

2. Méthode de dépistage de la maladie d'Alzheimer selon la revendication 1, dans laquelle l'échantillon de sang est du sérum ou du plasma.

3. Utilisation d'un réactif de diagnostic pour le dépistage de la maladie d'Alzheimer, comprenant, en tant que composants essentiels :

un peptide ß-amyloîde 1-42 devant être ajouté à un échantillon de sang, où le peptide ß-amyloïde 1-42 est un ß-amyloïde humain composé de 42 acides aminés et où le peptide ß-amyloïde 1-42 contient un site devant être clivé par une enzyme de dégradation ;
un anticorps qui reconnaît un site C-terminal du peptide ß-amyloïde 1-42 ; et
un anticorps qui reconnaît un site N-terminal du peptide ß-amyloïde 1-42,
dans laquelle l'anticorps qui reconnaît un site C-terminal du peptide ß-amyloïde 1-42 et l'anticorps qui reconnaît un site N-terminal du peptide ß-amyloïde 1-42 sont utilisés pour la mesure d'une quantité résiduelle du peptide ß-amyloïde 1-42 ajouté à l'échantillon de sang pour mesurer une activité de dégradation du peptide ß-amyloïde 1-42 dans l'échantillon de sang.

4. Utilisation selon la revendication 3, dans laquelle l'échantillon de sang est du sérum ou du plasma.

**FIG.1**

**FIG.2**

FIG.3

# FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0564946 A

### Non-patent literature cited in the description

- **TAKAKI Y et al.** *J. Biochem(Tokyo,* 2000, vol. 128 (6), 897-902
- **SHIROTANI K et al.** *J. Biol. Chem.,* 2001, vol. 276 (24), 21895-901
- **IWATA N et al.** *Science,* 2001, vol. 292 (5521), 1550-2
- **TAMAOKA A et al.** *J. Neurol. Sci,* 1997, vol. 148, 41-45
- **ANDREASEN N et al.** *Arch. Neurol.,* 1999, vol. 56, 673-680
- **GALASKO D et al.** *Arch. Neurol.,* 1998, vol. 55, 937-945
- **MOTTER R et al.** *Ann Neurol,* 1995, vol. 38, 643-648
- **TAMAOKA A et al.** *J. Neurol. Sci.,* 1996, vol. 141, 65-68
- **NAMBA et al.** Studies on New Immunoassay Method Using Electrochemiluminescence (ECL. *JJCLA,* 1996, vol. 21 (5
- *Nature,* 1975, vol. 256, 459-497